# EUROPEAN PATENT SPECIFICATION

(11) **EP 4 344 717 B1**
(45) Date of publication and mention of the grant of the patent: **12.08.2026**
(21) Application number: 22198272.1
(22) Date of filing: 28.09.2022
(51) Int. Cl.: A61M 5/34, A61M 39/20

(54) **SEALING CAP FOR A SYRINGE TIP**
VERSCHLUSSKAPPE FÜR EINE SPRITZENSPITZE
CAPUCHON D'ÉTANCHÉITÉ POUR UNE POINTE DE SERINGUE

(43) Date of publication of application: 03.04.2024
(73) Proprietor: Becton, Dickinson and Company, Franklin Lakes, NJ 07417-1880 (US)
(72) Inventor: DINC, Mirhac, 74960 Annecy (FR)
(74) Representative: Germain Maureau

(56) References cited:
- CN-U- 203 355 007
- KR-A- 20200 046 472
- US-A1- 2019 046 736
- US-A1- 2022 080 129

## Description

### BACKGROUND OF THE INVENTION

### Field of the Invention

The present invention is directed to a cap for covering the tip of a syringe to keep contaminants from contacting the tip of the syringe and/or entering the fluid passageway in the tip of the syringe, thereby potentially contaminating the medicament contained in the syringe.

### Description of Related Art

For prefilled syringes, the avoidance of the contamination of the syringe tip, needle, and/or contents of the syringe is imparted by a cap provided in an interference fit with the tip and covers the tip and/or needle. A seal is formed between the cap and the tip. The seal is necessary for and contributes to the overall container closure integrity (CCI) of the syringe assembly, which is a measurement of the overall capability of the syringe assembly to keep the medicament contained in the syringe from being contaminated.

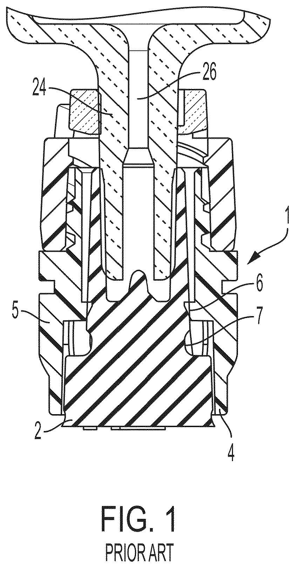

As shown in FIG. 1, prior art sealing caps 1 include a sealing insert 2 that forms the seal with the distal tip 24 of the syringe. The sealing insert 2 is surrounded by a housing 5. During placement of the sealing cap 1 on the syringe, the sealing insert 2 is pushed distally by the distal tip 24 of the syringe resulting in: (1) the upper portion 3 of the sealing insert 2 extending beyond the distal end 4 of the housing 5; (2) the engagement between a protrusion 6 in the housing 5 and a groove 7 in the sealing insert 2 intended to hold the sealing insert 2 within the housing 5 and ensure sealing contact of the sealing insert 2 with the distal tip 24 of the syringe being partially or completely disengaged; and (3) the stopper portion 8 of the sealing insert 2 intended to engage and seal the fluid passageway 26 of the distal tip 24 failing to fully engage and seal the fluid passageway 26. All of these factors negatively affect the overall sealing provided by the sealing cap 1. Further, US2019046736A1 discloses a syringe barrel, a syringe barrel manufacturing method, and a pre-filled syringe. KR20200046472A discloses a syringe cap with an anti-slip fastening structure. US2022080129A1 discloses a syringe cap assembly. CN203355007U discloses a kind of no-needle transfusion joint.

There is, therefore, a need for a sealing cap that does not suffer from these deficiencies when it is placed on the distal tip of the syringe.

### SUMMARY OF THE INVENTION

The present invention is directed to a sealing cap for a syringe provided according to claim 1.

The tip-engagement portion of the sealing insert may include an open proximal end corresponding to the proximal end of the sealing insert, a closed distal end, and a sidewall defining a cavity extending between the proximal end of the tip-engagement portion of the sealing insert and the distal end of the tip-engagement portion of the sealing insert and adapted and dimensioned to receive the distal tip of the syringe, thereby forming a seal between an inner surface of the sidewall of the tip-engagement portion of the sealing insert and an outer surface of the distal tip. A stopper protrusion may extend from the distal end of the tip-engagement portion of the sealing insert into the cavity of the tip-engagement portion, and is disposed and dimensioned to contact and at least partially extend into the passageway of the distal tip.

The housing-engagement portion of the sealing insert extends from the tip-engagement portion of the sealing insert in a distal direction and has a distal end that corresponds to the distal end of the sealing insert.

The locking rib may be spaced apart from the proximal end of the sealing insert such that a portion of the tip-engagement portion of the sealing insert extends proximally beyond the locking rib. The locking rib may have an outer peripheral face that is angled such that an outer diameter of a distal end of the locking rib is greater than an outer diameter of a proximal end of the locking rib and/or may have a trapezoidal cross-section.

An outer diameter of the housing-engagement portion of the sealing insert may be greater than an outer diameter of the tip-engagement portion. The locking rib may have an outer diameter that is intermediate between the outer diameter of the tip-engagement portion and the outer diameter of the housing-engagement portion of the sealing insert. An outer diameter of the connection portion of the housing may be less than an outer diameter of the insert-engagement portion of the housing. A diameter of the cavity of the housing that receives the tip-engagement portion of the sealing insert may be greater than the outer diameter of the tip-engagement portion, such that space is provided between the tip-engagement portion of the sealing insert and the housing. A diameter of the cavity of the housing at the annular protrusion of the housing may be approximately equal to a diameter of the sealing insert within the groove of the sealing insert.

The insert-engagement portion of the housing may have an open proximal end, a distal end, and a sidewall defining a cavity adapted and dimensioned to receive the housing-engagement portion of the sealing insert.

The annular protrusion of the housing may extend radially inwardly around the inner surface of the sidewall of the insert-engagement portion of the housing.

The connection portion of the housing extends proximally from the insert-engagement portion of the housing and comprises an open proximal end, an open distal end, and a sidewall defining a cavity adapted to receive at least a portion of the tip-engagement portion, and threads extend around an outer peripheral surface of the sidewall of the connection portion of the housing.

The annular protrusion of the housing may be spaced a distance from the distal end of the housing that is approximately equal to or greater than a distance between the groove of the sealing insert and the distal end of the sealing insert, and/or the locking rib may be spaced a distance from the groove of the sealing insert that is approximately equal to a distance between the annular protrusion of the housing and the proximal end of the housing.

A plurality of anti-rotation ribs may extend axially in a longitudinal direction along an outer peripheral surface of the housing-engagement portion of the sealing insert and/or a plurality of anti-rotation ribs may extend axially in a longitudinal direction along an inner peripheral surface of the insert-engagement portion of the housing. The anti-rotation ribs of the housing-engagement portion of the sealing insert may be received in spaces between the anti-rotation ribs of the insert-engagement portion of the housing. A proximal end of the anti-rotation ribs of the housing-engagement portion of the sealing insert and/or a distal end of the anti-rotation ribs of the housing-engagement portion of the sealing insert may be tapered or pointed.

A material of the housing may have a higher Young's modulus, higher strength, higher hardness, and/or lower elasticity than a material of the sealing insert. The sealing insert may be made from a material selected from the group consisting of polyethylene (PE), polypropylene (PP), and an elastomeric material, and the housing may be made from a material selected from the group consisting of polycarbonate (PC), polyoxymethylene (POM), polybutylene terephthalate (PBT), polypropylene (PP), polyethylene (PE), polyvinylchloride (PVC), polystyrene, polycarbonate, acrylonitrile butadiene styrene, styrene acrylonitrile, fiber-reinforced polymer, metal, and combinations thereof.

The present invention is also directed to a syringe comprising a syringe barrel defining a reservoir for containing a medicament; a distal tip having a passageway in fluid communication with the reservoir; and a sealing cap as discussed above. The sealing cap covers a distal end of the passageway and creates a seal between the sealing insert and an outer surface of the distal tip to keep air and contaminants from entering the cavity of the sealing cap and/or the passageway.

The connection portion of the housing may be threadedly engaged with a luer collar surrounding a proximal end of the distal tip such that the distal tip is received in a cavity of the tip-engagement portion of the sealing insert. The luer collar may be a separate component that is friction fitted onto the distal tip. The luer collar may comprise a proximal end adjacent a distal face of the syringe, an open distal end, and a sidewall spaced from and at least partially surrounding the distal tip, where an inner surface of the sidewall of the luer collar includes threads that are engaged by threads on an outer surface of the connection portion of the housing. A plurality of radially inward directed mounting projections may extend from the sidewall of the luer collar at the proximal end of the luer cover and frictionally engage the distal tip.

### BRIEF DESCRIPTION OF THE DRAWINGS

FIG. 1 is a cross-sectional view of a prior art sealing cap placed on a distal tip of a syringe;
FIG. 2 is an exploded side perspective view of a sealing cap and syringe according to the invention;
FIG. 3 is a cross-sectional view of an embodiment of a sealing cap according to the invention;
FIG. 4 is a cross-sectional view of the sealing insert of the sealing cap of FIG. 3;
FIG. 5 is a cross-sectional view of the housing of the sealing cap of FIG. 3;
FIG. 6 is a side perspective view of an embodiment of a sealing cap according to the invention;
FIG. 7 is a cross-sectional view of the sealing cap of FIG. 3 placed on a distal tip of a syringe having an integral luer collar;
FIG. 8 is a cross-sectional view of the sealing cap of FIG. 3 placed on a distal tip of a syringe having a separate luer collar; and
FIG. 9 is a cross-sectional view of the luer collar of FIG. 8.

### DESCRIPTION OF THE INVENTION

As used herein, any numerical values are expressed using a period as a decimal point and a comma as a thousand separator, for example, 1,234 would be one thousand two hundred thirty four, and 1.2 would be one and two tenths. Unless otherwise expressly specified, all numbers, such as those expressing values, ranges, amounts or percentages, may be read as if prefaced by the word "about", even if the term does not expressly appear. Any numerical range recited herein is intended to include all sub-ranges subsumed therein. For example, a range of "1 to 10" is intended to include any and all sub-ranges between and including the recited minimum value of 1 and the recited maximum value of 10, that is, all subranges beginning with a minimum value equal to or greater than 1 and ending with a maximum value equal to or less than 10, and all subranges in between, e.g., 1 to 6.3, or 5.5 to 10, or 2.7 to 6.1. Plural encompasses singular and vice versa. When ranges are given, any endpoints of those ranges and/or numbers within those ranges can be combined with the scope of the present invention. "Including", "such as", "for example" and like terms mean "including/such as/for example but not limited to".

For purposes of the description hereinafter, spatial orientation terms, as used, shall relate to the referenced embodiment as it is oriented in the accompanying drawings, figures, or otherwise described in the following detailed description. However, it is to be understood that the embodiments described hereinafter may assume many alternative variations and configurations. It is also to be understood that the specific components, devices, features, and operational sequences illustrated in the accompanying drawings, figures, or otherwise described herein are simply exemplary and should not be considered as limiting.

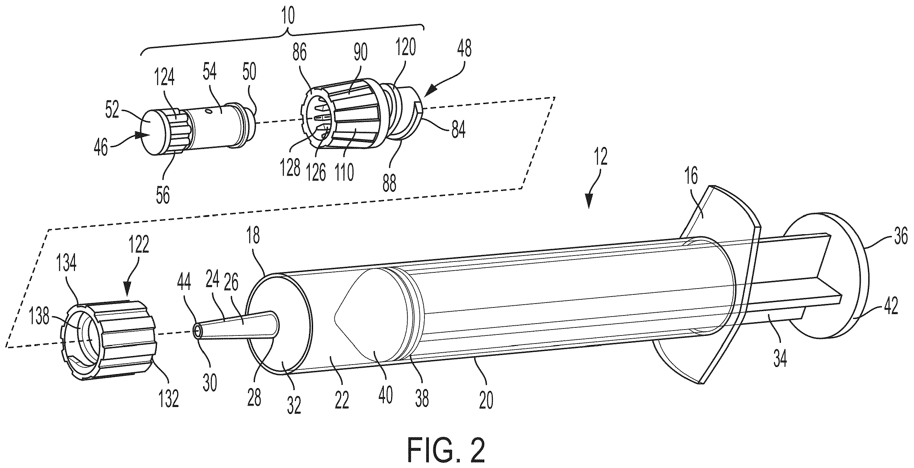

The present invention is directed to a sealing cap 10 for a syringe 12 and a syringe 12 including such a sealing cap 10 (FIG. 2).

As shown in FIG. 2, the inventive sealing cap 10 is adapted for use with a syringe 12 comprising a syringe barrel 14 having a proximal end 16, a distal end 18, and a sidewall 20 extending from the proximal end 16 to the distal end 18 and defining a reservoir 22 for containing a medicament. A distal tip 24 of the syringe 12 extends from the distal end 18 of the syringe barrel 14. A fluid passageway 26 extends through the distal tip 24 of the syringe 12 through which the medicament may be ejected from the reservoir 22.

The distal tip 24 of the syringe 12 may have an outer surface that is substantially cylindrical or the distal tip 24 of the syringe 12 may taper such that an outer diameter of the proximal end 28 of the distal tip 24 of the syringe 12 adjacent the distal end 18 of the syringe barrel 14 is larger than an outer diameter of the distal end 30 of the distal tip 24 of the syringe 12. The largest outer diameter of the distal tip 24 of the syringe 12 is smaller than an outer diameter of the syringe barrel 14, thereby forming a distal face 32 at the distal end 18 of the syringe barrel 14.

The syringe 12 may include a plunger rod 34 having a proximal end 36 and a distal end 38 with a stopper 40 extending from the distal end 38 and a pressing surface 42 extending from the proximal end 36. The stopper 40 forms a seal with the sidewall 20 of the syringe barrel 14 to contain the medicament within the reservoir 22, and the plunger rod 34 is moveable within the reservoir 22 of the syringe barrel 14 to eject the medicament from the reservoir 22 through the fluid passageway 26.

The inventive sealing cap 10 covers and seals a distal end 44 of the fluid passageway 26 and creates a seal with the distal tip 24 of the syringe 12 to keep air and contaminants from entering the fluid passageway 26 and thereby contaminating the medicament.

As shown in FIGS. 2-8, the sealing cap 10 comprises a sealing insert 46 that is received within a housing 48.

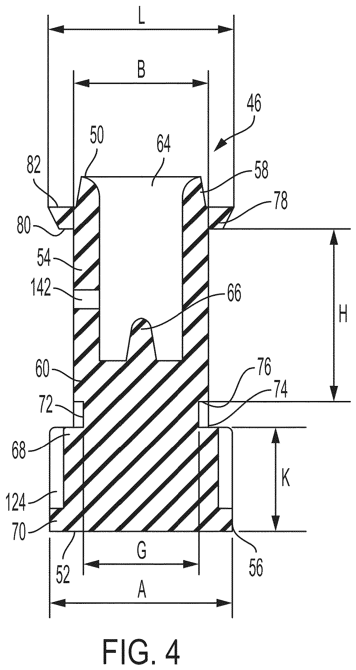

As shown in FIG. 4, the sealing insert 46 has an open proximal end 50, a closed distal end 52, a tip-engagement portion 54 at the proximal end 50, and a housing-engagement portion 56 at the distal end 52. The tip-engagement portion 54 comprises an open proximal end 58 corresponding to the proximal end 50 of the sealing insert 46, a closed distal end 60, and a sidewall 62 defining a cavity 64 extending between the proximal end 58 and the distal end 60 and adapted and dimensioned to receive the distal tip 24 of the syringe 12, thereby forming a seal between the inner surface of the sidewall 62 of the tip-engagement portion 54 of the sealing insert 46 and the outer surface of the distal tip 24 of the syringe 12. A stopper protrusion 66 extends from the distal end 60 of the tip-engagement portion 54 of the sealing insert 46 into the cavity 64 of the tip-engagement portion 54 of the sealing insert 46. The stopper protrusion 66 is disposed and dimensioned to contact and at least partially extend into the fluid passageway 26 of the distal tip 24 of the syringe 12.

The housing-engagement portion 56 extends in a distal direction and has a proximal end 68 and a distal end 70 that corresponds to the distal end 52 of the sealing insert 46.

The tip-engagement portion 54 of the sealing insert 46 is connected to the housing-engagement portion 56 of the sealing insert 46 by an annular groove 72 that extends around the periphery of the sealing insert 46. The groove 72 defines a distal face 74 at the distal end 60 of the tip-engagement portion 54 and a proximal face 76 at the proximal end 68 of the housing-engagement portion 56. The outer diameter A of the housing-engagement portion 56 of the sealing insert 46 may be greater than the outer diameter B of the tip-engagement portion 54 of the sealing insert 46.

An annular locking rib 78 extends around the outer periphery of the proximal end 58 of the tip-engagement portion 54 of the sealing insert 46. The locking rib 78 has an outer diameter L that is intermediate between the outer diameter B of the tip-engagement portion 54 of the sealing insert 46 and the outer diameter A of the housing-engagement portion 56 of the sealing insert 46. The locking rib 72 may be spaced apart from the proximal end 50 of the sealing insert 46 such that a portion of the tip-engagement portion 54 of the sealing insert 46 extends proximally beyond the locking rib 72. The locking rib 72 may have an outer peripheral face angled such that the outer diameter of the distal end 74 of the locking rib 72 is greater than the outer diameter of the proximal end 76 of the locking rib 72 and the locking rib 72 may have a trapezoidal cross-section.

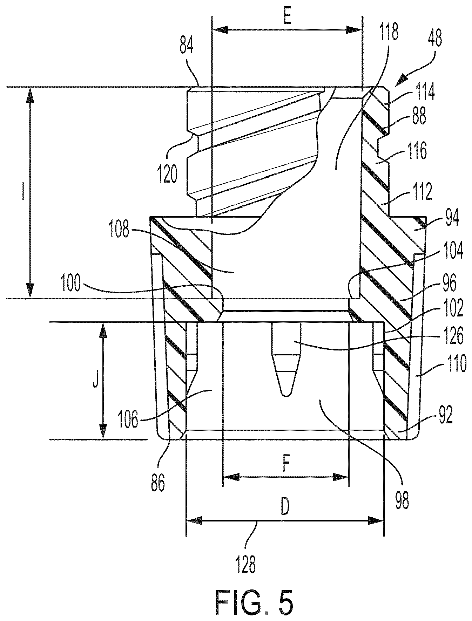

As shown in FIG. 5, the housing 48 comprises an open proximal end 84, a distal end 86, a connection portion 88 at the proximal end 84, and an insert-engagement portion 90 at the distal end 86. The housing 48 may be substantially cylindrical. The distal end 86 of the housing 48 may be open.

The insert-engagement portion 90 has an open proximal end 92, a distal end 94, and a sidewall 96 defining a cavity 98 extending between the proximal end 92 and the distal end 94 and adapted and dimensioned to receive the housing-engagement portion 56 of the sealing insert 46. The distal end 94 of the insert-engagement portion 90 may be open.

An annular protrusion 100 extends radially inwardly around the inner surface of the sidewall 96 of the insert-engagement portion 90 of the housing 48. The radial length of the distal face 102 of the protrusion 100 is larger than the radial length of the proximal face 104 of the protrusion 100, such that the diameter D of a distal portion 106 of the cavity 98 of the insert-engagement portion 90 of the housing 48 distal to the protrusion 100 is greater than the diameter E of a proximal portion 108 of the cavity 98 of the insert-engagement portion 90 of the housing 48 proximal to the protrusion 100.

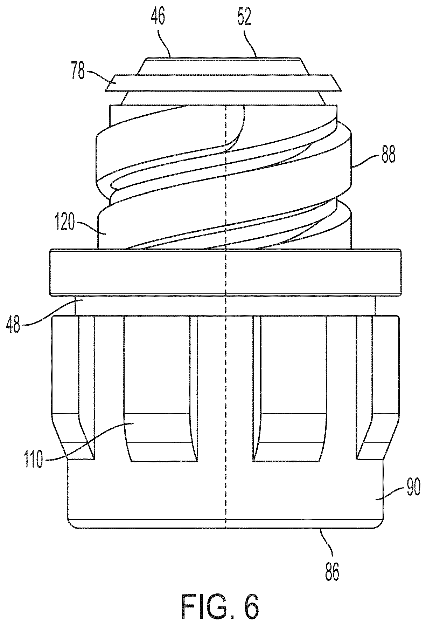

Optionally, grasping protrusions 110 may extend radially outward from an outer surface of the insert-engagement portion 90 of the housing 48 (FIG. 6). The grasping protrusions 110 are dimensioned and configured to facilitate manual gripping of the sealing cap 10 by a user during removal of the sealing cap 10 from the syringe 12. For example, the grasping protrusions 110 may be a plurality of ribs.

The connection portion 88 of the housing 48 extends proximally from the proximal end 92 of the insert-engagement portion 90 of the housing 48 and comprises an open proximal end 112, an open distal end 114, and a sidewall 116 extending from the proximal end 112 to the distal end 114 and defining a cavity 118. The diameter of the cavity 118 of the connection portion 88 of the housing 48 is the same as the diameter E of the proximal portion 108 of the cavity 98 of the insert-engagement portion 90 of the housing 48. Threads 120, which are engageable with the threads of a luer collar 122 surrounding the distal tip 24 of the syringe 12, extend around the outer peripheral surface of the sidewall 116 of the connection portion 88 of the housing 48.

The outer diameter of the connection portion 88 of the housing 48 is less than the outer diameter of the insert-engagement portion 90 of the housing 48

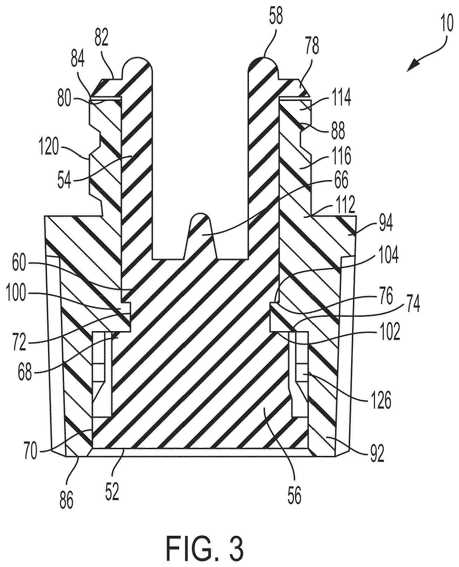

As shown in FIGS. 3, 7, and 8, the housing-engagement portion 56 of the sealing insert 46 is received within the distal portion 106 of the cavity 98 of the insert-engagement portion 90 of the housing 48, and the tip-engagement portion 54 of the sealing insert 46 is received within the proximal portion 108 of the cavity 98 of the insert-engagement portion 90 of the housing 48 and the cavity 118 of the connection portion 88 of the housing 48. The protrusion 100 of the housing 48 is received in the annular groove 72 of the sealing insert 46. The locking rib 78 of the sealing insert 46 and the proximal end 50 of the sealing insert 46 extend beyond the proximal end 84 of the housing 48 with the locking rib 78 of the sealing insert 46 engaging the proximal end 84 of the housing 48.

The diameter E of the proximal portion 108 of the cavity 98 of the insert-engagement portion 90 of the housing 48 and the cavity 118 of the connection portion 88 of the housing 48, which are equal, is greater than the outer diameter B of the tip-engagement portion 54 of the sealing insert 46, such that space is provided between the sealing insert 46 and the housing 48 to allow for expansion of the tip-engagement portion 54 of the sealing insert 46 when the sealing cap 10 is placed on the distal tip 24 of the syringe 12.

The protrusion 100 of the housing 48 is spaced a distance J from the distal end 86 of the housing 48 that is approximately equal to or greater than the distance K between the groove 72 of the sealing insert 46 and the distal end 52 of the sealing insert 46. The diameter F of the opening of the cavity 98 of the housing 48 at the protrusion 100 is approximately equal to the diameter G of the sealing insert 46 within the groove 72. The distal portion of the protrusion 100 of the housing 48 may be chamfered to facilitate deflection of the sealing insert 46 during assembly of the sealing cap 10 as will be discussed later.

The locking rib 78 of the sealing insert 46 is spaced a distance H from the groove 72 of the sealing insert 46 that is approximately equal to the distance I between the protrusion 100 of the housing 48 and the proximal end 84 of the housing 48.

The sidewall 96 of the insert-engagement portion 90 may optionally include openings (not shown) that allow for expansion of the housing-engagement portion 56 of the sealing insert 46 during assembly of the sealing cap 10, placement of the sealing cap 10 on the syringe 12, and/or removal of the sealing cap 10 from the syringe 12.

Optionally, a vent opening 142 may extend through the sidewall 62 of the tip-engagement portion 54 of the sealing insert 46 to reduce any vacuum that is created as the sealing insert 46 is removed from distal tip 24 of the syringe 12. When the sealing cap 10 is disposed on the distal tip 24 of the syringe 12, the vent opening 142 is blocked by the distal tip 24. As the sealing cap 10 is removed from the syringe 12 and the portion of the sealing insert 46 containing the vent opening 142 loses contact with the distal tip 24, the vent opening 142 allows air to enter the cavity 64 of the tip-engagement portion 54 of the sealing cap 10, minimizing any vacuum that may form as the sealing cap 10 is further removed from the syringe 12.

As shown in FIG. 1, prior art sealing caps 1 include a sealing insert 2 that forms the seal with the distal tip 24 of syringe. The sealing insert 2 is surrounded by a housing 5. During placement of the sealing cap 1 on the syringe: (1) the sealing insert 2 is pushed distally by the distal tip 24 of the syringe resulting in the upper portion 3 of the sealing insert 2 extending beyond the distal end 4 of the housing 5; (2) the engagement between a protrusion 6 in the housing 5 and a groove 7 in the sealing insert 2 intended to hold the sealing insert 2 within the housing 5 and ensure sealing contact of the sealing insert 2 with the distal tip 24 of the syringe being partially or completely disengaged; and (3) the stopper portion 8 of the sealing insert 7 intended to engage and seal the fluid passageway 26 of the distal tip 24 failing to fully engage and seal the fluid passageway 26. All of these factors negatively affect the overall sealing provided by the sealing cap 1.

The inventive sealing cap 10 corrects the deficiencies present in the prior art sealing cap 1. The combination of the engagement between the protrusion 100 of the housing 48 and the groove 72 of the sealing insert 46 and the engagement between the proximal end 84 of the housing 48 and the locking rib 78 of the sealing insert 46 acts to hold the sealing insert 46 within the housing 48 during placement of the sealing cap 10 on the distal tip 24 of the syringe 12 and to assure a good seal between the sealing insert 46 of the sealing cap 10 and the distal tip 24 of the syringe 12. The locking rib 78 keeps the sealing insert 46 from being pushed distally by the distal tip 24 of the syringe 12 and, thereby, keeps the insert-engagement portion 90 of the sealing insert 46 from extending beyond the distal end 86 of the housing 48, keeps the protrusion 100 of the housing 48 engaged with the groove 72 of the sealing insert 46, and keeps the stopper protrusion 66 of the sealing insert 46 fully engaged with and sealing the fluid passageway 26 of the distal tip 24 of the syringe 12.

Optionally, a plurality of anti-rotation ribs 124 may extend axially in a longitudinal direction along an outer peripheral surface of the housing-engagement portion 56 of the sealing insert 46, and/or a plurality of anti-rotation ribs 126 may extend axially in a longitudinal direction along an inner peripheral surface of the insert-engagement portion 90 of the housing 48 (FIGS. 2, 4, and 5). The anti-rotation ribs 124 of the sealing insert 46 may be received in the spaces between the anti-rotation ribs 126 of the housing 48 in order to keep the sealing insert 46 from rotating within the housing 48. The anti-rotation ribs 124 of the sealing insert 46 and/or anti-rotation ribs 126 of the housing 48 may be spaced equally around the peripheral surfaces of the housing-engagement portion 56 of the sealing insert and/or the connection portion 88 of the housing 48, respectively. For example, the anti-rotation ribs 124 and/or the anti-rotation ribs 126 may be spaced at approximately 45° from one another about the respective peripheral surface. The distal end 128 of the anti-rotation ribs 126 of the housing 48 and/or the proximal end 130 of the anti-rotation ribs 124 of the sealing insert 46 may be tapered or pointed to urge the anti-rotation ribs 124 of the sealing insert 46 into the spaces between the anti-rotation ribs 126 of the housing 48 during assembly of the sealing cap 10.

The sealing cap 10 is assembled by inserting the proximal end 50 of the sealing insert 46 into distal end 86 of the housing 48 and urging the sealing insert 46 in the proximal direction. The tip-engagement portion 54 of the sealing insert 46 engages the protrusion 100 of the housing 48 and is deflected radially inward. As the proximal movement of the sealing insert 46 is continued, the locking rib contacts the protrusion 100 of the housing 48 and the sealing insert 46 is further deflected radially inward until the locking rib 78 has passed the protrusion 100 of the housing 48. The angled outer peripheral face of the locking rib 78 assists in the deflection of the sealing insert 46 and the passage of the locking rib 78 past the protrusion 100 of the housing 48. After sufficient proximal movement of the sealing insert 46 with respect to the housing 48, the optional anti-rotation ribs 124 of the sealing insert 46 engage the anti-rotation ribs 126 of the housing 48. The pointed or tapered ends of anti-rotation ribs 124, 126 cause the sealing insert 46 to rotate slightly, such that anti-rotation ribs 124 of the sealing insert 46 align with the anti-rotation ribs 126 of the housing 48 and the sealing insert 46 can be further sealing inserted into the housing 48 with the anti-rotation ribs 124 of the sealing insert 46 positioned between the anti-rotation ribs 126 of the housing 48. Further advancement of the sealing insert 46 into the housing 48 causes the groove 72 of the sealing insert 46 to align with protrusion 100 of the housing 48 and the locking rib 78 of the sealing insert 46 to extend beyond the proximal end 84 of the housing 48. At this point, the sealing insert 46 resiliently returns toward an undeflected condition with the protrusion 100 of the housing 48 trapped in the groove 72 of the sealing insert 46 and the locking rib 78 of the sealing insert 46 engaging the proximal end 84 of the housing 48 to substantially prevent further axial movement between the sealing insert 46 and the housing 48. In this position, the interconnection of the anti-rotation ribs 124 of the sealing insert 46 and the anti-rotation ribs 126 of the housing 48 prevents or limits rotation of the sealing insert 46 relative to the housing 48.

As shown in FIGS. 2, 7, and 8, to attach the sealing cap 10 to the syringe 12, the connection portion 88 of the housing 48 of the sealing cap 10 is inserted into and threadedly engaged with a luer collar 122 surrounding a proximal end 28 of the distal tip 24. The distal tip 24 of the syringe 12 is received in the cavity 64 of the tip-engagement portion 54 of the sealing insert 46 with the inner surface of the sidewall 62 of the tip-engagement portion 54 of the sealing insert 46 creating a seal with at least a portion of the outer surface of the distal tip 24 and the stopper protrusion 100 of the tip-engagement portion 54 of the sealing insert 46 extending into and sealing the fluid passageway 26 of the distal tip 24.

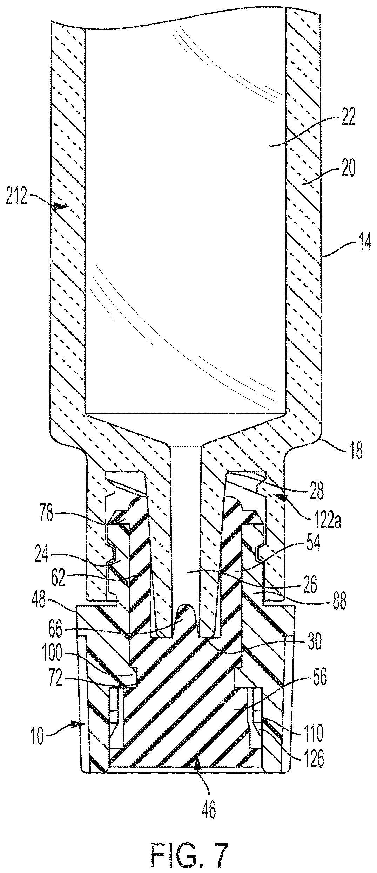

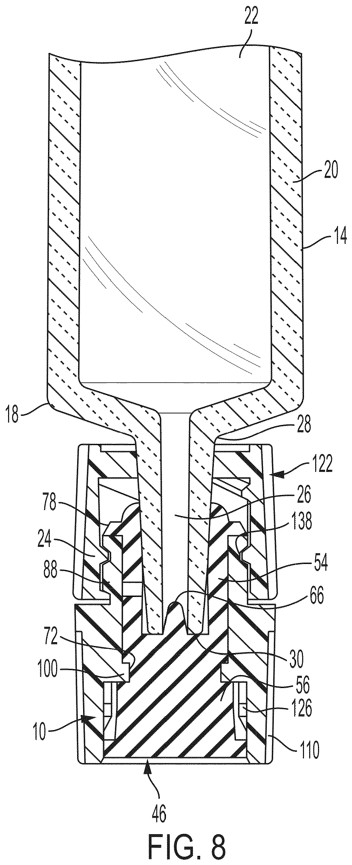

The luer collar 122 may be a separate component that is friction fitted onto the distal tip 24 of the syringe 12 (FIG. 8) or may be integrally molded with the distal tip 24 of the syringe 212 (FIG. 7). The luer collar 122 comprises a proximal end 132 attached to the distal face 32 of the syringe 12, an open distal end 134, and a sidewall 136 extending from the proximal end 132 to the distal end 134. The sidewall 136 of the luer collar 122 is spaced from and at least partially surrounds the distal tip 24 of the syringe 12 and an inner surface of the sidewall 136 of the luer collar 122 includes threads 138 that are engaged by the threads 120 of the connection portion 88 of the housing 48 of the sealing cap 10. The sidewall 136 of the luer collar 122 is concentric with the distal tip 24.

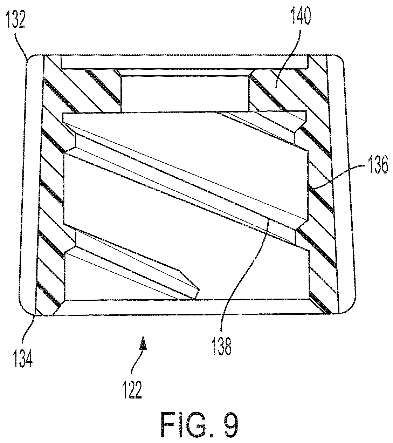

If the luer collar 122 is separate from the distal tip 24, a plurality of radially inward directed mounting projections 140 extend from the inner surface of the sidewall 136 of the luer collar 122 at the proximal end 132 of the luer collar 122 (FIG. 9). As the proximal end 132 of the luer collar 122 is urged in a proximal direction over the distal tip 24 of the syringe 12, the mounting projections 140 deflect slightly and frictionally engage the distal tip 24 to resist separation of luer collar 122 from the syringe 12.

Unintended separation of sealing cap 10 from the distal tip 24 is substantially prevented by the threaded engagement of sealing cap 10 with the luer collar 122. Further, the threaded engagement of sealing cap 10 with the luer collar 122 and the simultaneous engagement of the housing 48 with the sealing insert 46 positively prevent inadvertent breaking of the seal between the sealing insert 46 and the distal tip 24. However, the distal tip 24 of the syringe 12 can be readily accessed by rotating the sealing cap 10 to disengage the threads 120 of the sealing cap 10 from the threads 138 of the luer collar 122. Engagement of the optional anti-rotation ribs 124 of the housing-engagement portion 56 of the sealing insert 46 with the anti-rotation ribs 126 of the insert-engagement portion of the housing 48 ensures that the sealing insert 46 will rotate with the housing 48. Engagement of the groove 72 of the sealing insert 46 with the protrusion 100 of the housing 48 and the locking rib 78 of the sealing insert 46 with the proximal end 84 of the housing 48 ensures that the sealing insert 46 will move axially in response to the disengagement of the connection portion 88 of the housing 48 from the luer collar 122. Further, the locking rib 78 of the sealing insert 46 blocks and prevents the sealing insert from rolling and/or displacement, during assembly, during placement on the syringe 12 and/or removal from the syringe 12.

The sealing insert 46 may be made from any suitable material that is sufficiently flexible to compress and conform to the outer surface of the distal tip 24 of the syringe 12 to create a seal between the sealing insert 46 and the outer surface of the distal tip 24 of the syringe 12 that prevents air and contaminants from entering the fluid passageway 26 of the distal tip 24 of the syringe 12. For example, the sealing insert 46 may be made from polyethylene (PE), polypropylene (PP), or an elastomeric material, for example a rubber material such as styrene butadiene rubber, an isoprene bromobuytyl blend, or a thermoplastic elastomer. Suitable materials are inert to the medical solution to be filled into the medical container, have sufficient heat resistance to undergo the necessary sterilization procedures, and are sufficiently flexible to grip and release the distal tip 24 of the syringe 12. The sealing insert 46 may be manufactured by a molding process such as injection molding.

The material of the housing 48 has higher Young's modulus (stiffness), higher strength, higher hardness, and/or lower elasticity than the sealing insert 46. For example, the sealing insert 46 may be an elastomer having a Young's modulus of 0.0001 to 0.1 GPa, and the housing 48 may be a thermoplastic having a Young's modulus of 1-10 GPa.

The housing 48 may be made from any suitable material that provides sufficient compressive contact force on the sealing insert 46 to maintain a continuous seal between the sealing insert 46 and the outer surface of the distal tip 24 of the syringe 12 that prevents air and contaminants from entering the fluid passageway 26 of the distal tip 24 of the syringe 12. For example, the housing 48 may be made from a thermoplastic, such as, polycarbonate (PC), polyoxymethylene (POM), polybutylene terephthalate (PBT), polypropylene (PP), polyethylene (PE), polyvinylchloride (PVC), polystyrene, acrylonitrile butadiene styrene, styrene acrylonitrile, a fiber-reinforced polymer, a metal, or a combination thereof. The housing 48 may be made of polypropylene, as it is an easily moldable, strong and lightweight plastic material. Polypropylene further has the advantages of being highly impermeable to liquids and inert with respect to many chemicals, which prevents degradation of the housing 48 when it gets into contact with a medical solution. The housing 48 may be made of a transparent material which allows for verifying the correct assembly of the housing 48 and the sealing insert 46.

Whereas particular aspects of this invention have been described above for purposes of illustration, it will be evident to those skilled in the art that numerous variations of the details of the present invention may be made without departing from the invention as defined by the appended claims.

## Claims

1. A sealing cap (10) for a syringe (12) comprising a syringe barrel (14) defining a reservoir (22) for containing a medicament and a distal tip (24) having a passageway (26) in fluid communication with the reservoir (22), the sealing cap (10) comprising:
a housing (48) having an open proximal end (84), a distal end (86), and a sidewall (96, 116) extending from the proximal end (84) to the distal end (86) and defining a cavity (98, 118); and
a sealing insert (46) received within the cavity (98, 118) of the housing (48),
wherein the housing (48) comprises a connection portion (88) and an insert-engagement portion (90), and the sealing insert (46) comprises a tip-engagement portion (54), and a housing-engagement portion (56),
the housing-engagement portion (56) of the sealing insert (46) is received within the insert-engagement portion (90) of the housing (48), and the tip-engagement portion (54) of the sealing insert (46) is received within the connection portion (88) of the housing (48), and
an annular protrusion (100) extends radially inwardly from the sidewall (96, 116) of the housing (48) and is received in an annular groove (72) positioned between and connecting the housing-engagement portion (56) of the sealing insert (46) and the tip-engagement portion (54) of the sealing insert (46),
**characterized in that** an annular locking rib (78) extends around an outer periphery of a proximal end (50) of the sealing insert (46) and is positioned proximally beyond the proximal end (84) of the housing (48), and
during placement of the sealing cap (10) on the distal tip (24) of the syringe (12), an engagement between the locking rib (78) of the sealing insert (46) and the proximal end (84) of the housing (48) is configured to keep the sealing insert (46) from being moved in a distal direction to keep the annular protrusion (100) of the housing (48) engaged with the annular groove (72) of the sealing insert (46).

2. The sealing cap (10) of claim 1, wherein the tip-engagement portion (54) comprises an open proximal end (58) corresponding to the proximal end (50) of the sealing insert (46), a closed distal end (60), and a sidewall (62) defining a cavity (64) extending between the proximal end (58) of the tip-engagement portion (54) and the distal end (60) of the tip-engagement portion (54) and adapted and dimensioned to receive the distal tip (24) of the syringe (12), thereby forming a seal between an inner surface of the sidewall (62) of the tip-engagement portion (54) and an outer surface of the distal tip (24).

3. The sealing cap (10) of claim 2, wherein a stopper protrusion (66) extends from the distal end (60) of the tip-engagement portion (54) into the cavity (64) of the tip-engagement portion (54), and is disposed and dimensioned to contact and at least partially extend into the passageway (26) of the distal tip (24).

4. The sealing cap (10) of any of claims 1-3, wherein the housing-engagement portion (56) extends from the tip-engagement portion (54) of the sealing insert (46) in a distal direction and has a distal end (70) that corresponds to a distal end (52) of the sealing insert (46).

5. The sealing cap (10) of any of claims 1-4, wherein the locking rib (78) is spaced apart from the proximal end (50) of the sealing insert (46) such that a portion of the tip-engagement portion (54) extends proximally beyond the locking rib (78).

6. The sealing cap (10) of any of claims 1-5, wherein the locking rib (78) has an outer peripheral face that is angled.

7. The sealing cap (10) of any of claims 1-6, wherein the locking rib (78) has a trapezoidal cross-section.

8. The sealing cap (10) of any of claims 1-7, wherein an outer diameter (A) of the housing-engagement portion (56) is greater than an outer diameter (B) of the tip-engagement portion (54), and the locking rib (78) has an outer diameter (L) that is intermediate between the outer diameter (B) of the tip-engagement portion (54) and the outer diameter (A) of the housing-engagement portion (56).

9. The sealing cap (10) of any of claims 1-8, wherein the insert-engagement portion (90) has an open proximal end (92), a distal end (94), and a sidewall (96) defining a cavity (98) adapted and dimensioned to receive the housing-engagement portion (56).

10. The sealing cap (10) of any of claims 1-9, wherein the annular protrusion (100) extends radially inwardly around the inner surface of the sidewall (96) of the insert-engagement portion (90).

11. The sealing cap (10) of any of claims 1-10, wherein the connection portion (88) extends proximally from the insert-engagement portion (90) and comprises an open proximal end (112), an open distal end (114), a sidewall (116) defining a cavity (118) adapted to receive at least a portion of the tip-engagement portion (54), and threads (120) extending around an outer peripheral surface of the sidewall (116) of the connection portion (88).

12. The sealing cap (10) of any of claims 1-11, wherein an outer diameter of the connection portion (88) is less than an outer diameter of the insert-engagement portion (90).

13. The sealing cap (10) of any of claims 1-12, wherein a diameter of the cavity (98) of the housing (48) that receives the tip-engagement portion (54) is greater than the outer diameter of the tip-engagement portion (54), such that space is provided between the tip-engagement portion (54) and the housing (48).

14. The sealing cap (10) of any of claims 1-13, wherein the annular protrusion (100) is spaced a distance (J) from the distal end (86) of the housing (48) that is approximately equal to or greater than a distance (K) between the annular groove (72) and a distal end (52) of the sealing insert (46).

15. The sealing cap (10) of any of claims 1-14, wherein a diameter (F) of the cavity (118) of the housing (48) at the annular protrusion (100) is approximately equal to a diameter (G) of the sealing insert (46) within the annular groove (72).

16. The sealing cap (10) of any of claims 1-15, wherein the locking rib (78) is spaced a distance (H) from the annular groove (72) that is approximately equal to a distance (I) between the annular protrusion (100) and the proximal end (84) of the housing (48).

17. The sealing cap (10) of any of claims 1-16, further comprising a plurality of anti-rotation ribs (124) extending axially in a longitudinal direction along an outer peripheral surface of the housing-engagement portion (56) and/or a plurality of anti-rotation ribs (126) extending axially in a longitudinal direction along an inner peripheral surface of the insert-engagement portion (90).

18. The sealing cap (10) of claim 17, wherein the anti-rotation ribs (124) of the housing-engagement portion (56) are received in spaces between the anti-rotation ribs (126) of the insert-engagement portion (90).

19. The sealing cap (10) of any of claims 17-18, wherein a proximal end (130) of the anti-rotation ribs (124) of the housing-engagement portion (56) and/or a distal end (128) of the anti-rotation ribs (126) of the insert-engagement portion (90) are tapered or pointed.

20. The sealing cap (10) of any of claims 1-19, wherein a material of the housing (48) has a higher Young's modulus, higher strength, higher hardness, and/or lower elasticity than a material of the sealing insert (46).

21. The sealing cap (10) of any of claims 1-20, wherein the sealing insert (46) is made from a material selected from the group consisting of polyethylene (PE), polypropylene (PP), an elastomeric material, and the housing (48) is made from a material selected from the group consisting of polycarbonate (PC), polyoxymethylene (POM), polybutylene terephthalate (PBT), polypropylene (PP), polyethylene (PE), polyvinylchloride (PVC), polystyrene, acrylonitrile butadiene styrene, styrene acrylonitrile, fiber-reinforced polymer, metal, and combinations thereof.

22. A syringe (12) comprising:
a syringe barrel (14) defining a reservoir (22) for containing a medicament;
a distal tip (24) having a passageway (36) in fluid communication with the reservoir (22); and
a sealing cap (10) according to any of claims 1-21,
wherein the sealing cap (10) is configured to cover a distal end (44) of the passageway (26) and to create a seal between the sealing insert (46) and an outer surface of the distal tip (24) to keep air and contaminants from entering the cavity (98, 118) of the sealing cap (10) and/or the passageway (26).

23. The syringe (12) of claim 22, wherein the connection portion (88) is threadedly engaged with a luer collar (122) surrounding a proximal end (28) of the distal tip (24), and the distal tip (24) is received in a cavity (64) of the tip-engagement portion (54) of the sealing insert (46).

24. The syringe (12) of claim 23, wherein the luer collar (122) is a separate component that is friction fitted onto the distal tip (24).

25. The syringe (12) of claim 24, wherein the luer collar (122) comprises a proximal end (132) adjacent a distal face of the syringe (12), an open distal end (134), and a sidewall (136) spaced from and at least partially surrounding the distal tip (24), and an inner surface of the sidewall (136) of the luer collar (122) includes threads (138) that are engaged by threads (120) on an outer surface of the connection portion (88) and a plurality of radially inward directed mounting projections (140) extend from the sidewall (136) of the luer collar (122) at the proximal end (132) of the luer collar (122) and frictionally engage the distal tip (24).

## Patentansprüche

1. Verschlusskappe (10) für eine Spritze (12), umfassend einen Spritzenzylinder (14), der ein Reservoir (22) zum Aufbewahren eines Arzneimittels definiert, und eine distale Spitze (24) mit einem Durchgang (26) in Fluidkommunikation mit dem Reservoir (22), wobei die Verschlusskappe (10) umfasst:
ein Gehäuse (48) mit einem offenen proximalen Ende (84), einem distalen Ende (86) und einer Seitenwand (96, 116), die sich vom proximalen Ende (84) zum distalen Ende (86) erstreckt und einen Hohlraum (98, 118) definiert; und
einen Dichtungseinsatz (46), der in dem Hohlraum (98, 118) des Gehäuses (48) aufgenommen ist,
wobei das Gehäuse (48) einen Verbindungsabschnitt (88) und einen Einsatz-Eingriffsabschnitt (90) umfasst, und der Dichtungseinsatz (46) einen Spitzen-Eingriffsabschnitt (54) und einen Gehäuse-Eingriffsabschnitt (56) umfasst,
der Gehäuse-Eingriffsabschnitt (56) des Dichtungseinsatzes (46) innerhalb des Einsatz-Eingriffsabschnitts (90) des Gehäuses (48) aufgenommen ist, und der Spitzen-Eingriffsabschnitt (54) des Dichtungseinsatzes (46) innerhalb des Verbindungsabschnitts (88) des Gehäuses (48) aufgenommen ist, und
einen ringförmigen Vorsprung (100), der sich radial von der Seitenwand (96, 116) des Gehäuses (48) nach innen erstreckt und in einer ringförmigen Nut (72) aufgenommen ist, die zwischen dem Gehäuse-Eingriffsabschnitt (56) des Dichtungseinsatzes (46) und dem Spitzen-Eingriffsabschnitt (54) des Dichtungseinsatzes (46) positioniert ist und diese verbindet,
**dadurch gekennzeichnet, dass** sich eine ringförmige Verriegelungsrippe (78) um einen äußeren Umfang eines proximalen Endes (50) des Dichtungseinsatzes (46) herum erstreckt und proximal über das proximale Ende (84) des Gehäuses (48) hinaus positioniert ist, und
beim Aufsetzen der Verschlusskappe (10) auf die distale Spitze (24) der Spritze (12) ein Eingriff zwischen der Verriegelungsrippe (78) des Dichtungseinsatzes (46) und dem proximalen Ende (84) des Gehäuses (48) eingerichtet ist, um zu verhindern, dass sich der Dichtungseinsatz (46) in eine distale Richtung bewegt, um den ringförmigen Vorsprung (100) des Gehäuses (48) mit der ringförmigen Nut (72) des Dichtungseinsatzes (46) in Eingriff zu halten.

2. Verschlusskappe (10) nach Anspruch 1, wobei der Spitzen-Eingriffsabschnitt (54) ein offenes proximales Ende (58), das dem proximalen Ende (50) des Dichtungseinsatzes (46) entspricht, ein geschlossenes distales Ende (60) und eine Seitenwand (62) umfasst, die einen Hohlraum (64) definiert, der sich zwischen dem proximalen Ende (58) des Spitzen-Eingriffsabschnitts (54) und dem distalen Ende (60) des Spitzen-Eingriffsabschnitts (54) erstreckt und so angepasst und dimensioniert ist, dass er die distale Spitze (24) der Spritze (12) aufnimmt, wodurch eine Abdichtung zwischen einer Innenfläche der Seitenwand (62) des Spitzen-Eingriffsabschnitts (54) und einer Außenfläche der distalen Spitze (24) gebildet wird.

3. Verschlusskappe (10) nach Anspruch 2, wobei sich ein Stopfenvorsprung (66) vom distalen Ende (60) des Spitzen-Eingriffsabschnitts (54) in den Hohlraum (64) des Spitzen-Eingriffsabschnitts (54) erstreckt und so angeordnet und dimensioniert ist, dass er den Durchgang (26) der distalen Spitze (24) berührt und sich mindestens teilweise in diesen erstreckt.

4. Verschlusskappe (10) nach einem der Ansprüche 1 bis 3, wobei sich der Gehäuse-Eingriffsabschnitt (56) vom Spitzen-Eingriffsabschnitt (54) des Dichtungseinsatzes (46) in einer distalen Richtung erstreckt und ein distales Ende (70) aufweist, das einem distalen Ende (52) des Dichtungseinsatzes (46) entspricht.

5. Verschlusskappe (10) nach einem der Ansprüche 1 bis 4, wobei die Verriegelungsrippe (78) von dem proximalen Ende (50) des Dichtungseinsatzes (46) so beabstandet ist, dass sich ein Abschnitt des Spitzen-Eingriffsabschnitts (54) proximal über die Verriegelungsrippe (78) hinaus erstreckt.

6. Verschlusskappe (10) nach einem der Ansprüche 1 bis 5, wobei die Verriegelungsrippe (78) eine äußere Umfangsfläche aufweist, die abgewinkelt ist.

7. Verschlusskappe (10) nach einem der Ansprüche 1 bis 6, wobei die Verriegelungsrippe (78) einen trapezförmigen Querschnitt aufweist.

8. Verschlusskappe (10) nach einem der Ansprüche 1 bis 7, wobei ein Außendurchmesser (A) des Gehäuse-Eingriffsabschnitts (56) größer ist als ein Außendurchmesser (B) des Spitzen-Eingriffsabschnitts (54), und die Verriegelungsrippe (78) einen Außendurchmesser (L) aufweist, der zwischen dem Außendurchmesser (B) des Spitzen-Eingriffsabschnitts (54) und dem Außendurchmesser (A) des Gehäuse-Eingriffsabschnitts (56) liegt.

9. Verschlusskappe (10) nach einem der Ansprüche 1 bis 8, wobei der Einsatz-Eingriffsabschnitt (90) ein offenes proximales Ende (92), ein distales Ende (94) und eine Seitenwand (96) aufweist, die einen Hohlraum (98) definiert, der so angepasst und dimensioniert ist, dass er den Gehäuse-Eingriffsabschnitt (56) aufnimmt.

10. Verschlusskappe (10) nach einem der Ansprüche 1 bis 9, wobei sich der ringförmige Vorsprung (100) um die Innenfläche der Seitenwand (96) des Einsatz-Eingriffsabschnitts (90) herum radial nach innen erstreckt.

11. Verschlusskappe (10) nach einem der Ansprüche 1 bis 10, wobei sich der Verbindungsabschnitt (88) proximal vom Einsatz-Eingriffsabschnitt (90) erstreckt und ein offenes proximales Ende (112), ein offenes distales Ende (114), eine Seitenwand (116), die einen Hohlraum (118) definiert, der so angepasst ist, dass er mindestens einen Abschnitt des Spitzen-Eingriffsabschnitts (54) aufnimmt, und Gewinde (120) umfasst, die sich um eine äußere Umfangsfläche der Seitenwand (116) des Verbindungsbschnitts (88) herum erstrecken.

12. Verschlusskappe (10) nach einem der Ansprüche 1 bis 11, wobei ein Außendurchmesser des Verbindungsabschnitts (88) kleiner als ein Außendurchmesser des Einsatz-Eingriffsabschnitts (90) ist.

13. Verschlusskappe (10) nach einem der Ansprüche 1 bis 12, wobei ein Durchmesser des Hohlraums (98) des Gehäuses (48), der den Spitzen-Eingriffsabschnitt (54) aufnimmt, größer ist als der Außendurchmesser des Spitzen-Eingriffsabschnitts (54), so dass zwischen dem Spitzen-Eingriffsabschnitt (54) und dem Gehäuse (48) ein Raum vorgesehen ist.

14. Verschlusskappe (10) nach einem der Ansprüche 1 bis 13, wobei der ringförmige Vorsprung (100) in einem Abstand (J) vom distalen Ende (86) des Gehäuses (48) beabstandet ist, der ungefähr gleich oder größer als ein Abstand (K) zwischen der ringförmigen Nut (72) und einem distalen Ende (52) des Dichtungseinsatzes (46) ist.

15. Verschlusskappe (10) nach einem der Ansprüche 1 bis 14, wobei ein Durchmesser (F) des Hohlraums (118) des Gehäuses (48) am ringförmigen Vorsprung (100) etwa gleich einem Durchmesser (G) des Dichtungseinsatzes (46) innerhalb der ringförmigen Nut (72) ist.

16. Verschlusskappe (10) nach einem der Ansprüche 1 bis 15, wobei die Verriegelungsrippe (78) in einem Abstand (H) von der ringförmigen Nut (72) beabstandet ist, der ungefähr gleich einem Abstand (I) zwischen dem ringförmigen Vorsprung (100) und dem proximalen Ende (84) des Gehäuses (48) ist.

17. Verschlusskappe (10) nach einem der Ansprüche 1 bis 16, ferner umfassend eine Vielzahl von Antirotationsrippen (124), die sich axial in einer Längsrichtung entlang einer äußeren Umfangsfläche des Gehäuse-Eingriffsabschnitts (56) erstrecken, und/oder eine Vielzahl von Antirotationsrippen (126), die sich axial in einer Längsrichtung entlang einer inneren Umfangsfläche des Einsatz-Eingriffsabschnitts (90) erstrecken.

18. Verschlusskappe (10) nach Anspruch 17, wobei die Antirotationsrippen (124) des Gehäuse-Eingriffsabschnitts (56) in Räumen zwischen den Antirotationsrippen (126) des Einsatz-Eingriffsabschnitts (90) aufgenommen sind.

19. Verschlusskappe (10) nach einem der Ansprüche 17 bis 18, wobei ein proximales Ende (130) der Antirotationsrippen (124) des Gehäuse-Eingriffsabschnitts (56) und/oder ein distales Ende (128) der Antirotationsrippen (126) des Einsatz-Eingriffsabschnitts (90) konisch oder spitz zulaufend ist.

20. Verschlusskappe (10) nach einem der Ansprüche 1 bis 19, wobei ein Material des Gehäuses (48) einen höheren Youngschen Modul, eine höhere Festigkeit, eine höhere Härte und/oder eine geringere Elastizität aufweist als ein Material des Dichtungseinsatzes (46).

21. Verschlusskappe (10) nach einem der Ansprüche 1 bis 20, wobei der Dichtungseinsatz (46) aus einem Material hergestellt ist, das aus der Gruppe ausgewählt ist, die aus Polyethylen (PE), Polypropylen (PP) und einem elastomeren Material besteht, und das Gehäuse (48) aus einem Material hergestellt ist, das aus der Gruppe ausgewählt ist, die aus Polycarbonat (PC), Polyoxymethylen (POM), Polybutylenterephthalat (PBT), Polypropylen (PP), Polyethylen (PE), Polyvinylchlorid (PVC), Polystyrol, Acrylnitril-Butadien-Styrol, Styrol-Acrylnitril, faserverstärktem Polymer, Metall und Kombinationen davon besteht.

22. Spritze (12), umfassend:
einen Spritzenzylinder (14), der ein Reservoir (22) zum Aufbewahren eines Arzneimittels definiert;
eine distale Spitze (24) mit einem Durchgang (36) in Fluidkommunikation mit dem Reservoir (22); und
eine Verschlusskappe (10) nach einem der Ansprüche 1-21,
wobei die Verschlusskappe (10) so eingerichtet ist, dass sie ein distales Ende (44) des Durchgangs (26) abdeckt und eine Abdichtung zwischen dem Dichtungseinsatz (46) und einer Außenfläche der distalen Spitze (24) erzeugt, um zu verhindern, dass Luft und Verunreinigungen in den Hohlraum (98, 118) der Verschlusskappe (10) und/oder den Durchgang (26) gelangen.

23. Spritze (12) nach Anspruch 22, wobei der Verbindungsabschnitt (88) mit einem Luer-Kragen (122) verschraubt ist, der ein proximales Ende (28) der distalen Spitze (24) umgibt, und die distale Spitze (24) in einem Hohlraum (64) des Spitzen-Eingriffsabschnitts (54) des Dichtungseinsatzes (46) aufgenommen ist.

24. Spritze (12) nach Anspruch 23, wobei der Luer-Kragen (122) eine separate Komponente ist, die reibschlüssig an der distalen Spitze (24) angebracht ist.

25. Spritze (12) nach Anspruch 24, wobei der Luer-Kragen (122) ein proximales Ende (132), das an eine distale Fläche der Spritze (12) angrenzt, ein offenes distales Ende (134) und eine Seitenwand (136) umfasst, die von der distalen Spitze (24) beabstandet ist und diese mindestens teilweise umgibt, und eine Innenfläche der Seitenwand (136) des Luer-Kragens (122) Gewinde (138) enthält, die durch Gewinde (120) mit einer Außenfläche des Verbindungsabschnitts (88) in Eingriff steht, und eine Vielzahl von radial nach innen gerichteten Montagevorsprüngen (140) sich von der Seitenwand (136) des Luer-Kragens (122) am proximalen Ende (132) des Luer-Kragens (122) erstrecken und die distale Spitze (24) reibschlüssig in Eingriff nehmen.

## Revendications

1. Capuchon d'étanchéité (10) pour une seringue (12) comprenant un cylindre de seringue (14) définissant un réservoir (22) pour contenir un médicament et une pointe distale (24) ayant un passage (26) en communication fluidique avec le réservoir (22), le capuchon d'étanchéité (10) comprenant :
un boîtier (48) ayant une extrémité proximale ouverte (84), une extrémité distale (86) et une paroi latérale (96, 116) s'étendant de l'extrémité proximale (84) à l'extrémité distale (86) et définissant une cavité (98, 118) ; et
un insert d'étanchéité (46) reçu à l'intérieur de la cavité (98, 118) du boîtier (48),
dans lequel le boîtier (48) comprend une partie de connexion (88) et une partie d'engagement d'insert (90), et l'insert d'étanchéité (46) comprend une partie d'engagement de pointe (54), et une partie d'engagement de boîtier (56),
la partie d'engagement de boîtier (56) de l'insert d'étanchéité (46) est reçue à l'intérieur de la partie d'engagement d'insert (90) du boîtier (48), et la partie d'engagement de pointe (54) de l'insert d'étanchéité (46) est reçue à l'intérieur de la partie de connexion (88) du boîtier (48), et
une saillie annulaire (100) s'étend radialement vers l'intérieur depuis la paroi latérale (96, 116) du boîtier (48) et est reçue dans une rainure annulaire (72) positionnée entre la partie d'engagement de boîtier (56) de l'insert d'étanchéité (46) et la partie d'engagement de pointe (54) de l'insert d'étanchéité (46) et reliant celles-ci,
**caractérisé en ce qu'**une nervure de verrouillage annulaire (78) s'étend autour d'une périphérie externe d'une extrémité proximale (50) de l'insert d'étanchéité (46) et est positionnée de manière proximale au-delà de l'extrémité proximale (84) du boîtier (48), et
pendant le placement du capuchon d'étanchéité (10) sur la pointe distale (24) de la seringue (12), un engagement entre la nervure de verrouillage (78) de l'insert d'étanchéité (46) et l'extrémité proximale (84) du boîtier (48) est configuré pour empêcher l'insert d'étanchéité (46) de se déplacer dans une direction distale afin de maintenir la saillie annulaire (100) du boîtier (48) engagée avec la rainure annulaire (72) de l'insert d'étanchéité (46).

2. Capuchon d'étanchéité (10) selon la revendication 1, dans lequel la partie d'engagement de pointe (54) comprend une extrémité proximale ouverte (58) correspondant à l'extrémité proximale (50) de l'insert d'étanchéité (46), une extrémité distale fermée (60), et une paroi latérale (62) définissant une cavité (64) s'étendant entre l'extrémité proximale (58) de la partie d'engagement de pointe (54) et l'extrémité distale (60) de la partie d'engagement de pointe (54), et adaptée et dimensionnée pour recevoir la pointe distale (24) de la seringue (12), formant ainsi une étanchéité entre une surface interne de la paroi latérale (62) de la partie d'engagement de pointe (54) et une surface externe de la pointe distale (24).

3. Capuchon d'étanchéité (10) selon la revendication 2, dans lequel une saillie de butée (66) s'étend depuis l'extrémité distale (60) de la partie d'engagement de pointe (54) dans la cavité (64) de la partie d'engagement de pointe (54), et est disposée et dimensionnée de manière à entrer en contact avec le passage (26) de la pointe distale (24) et à s'étendre au moins partiellement dans celui-ci.

4. Capuchon d'étanchéité (10) selon l'une des revendications 1 à 3, dans lequel la partie d'engagement de boîtier (56) s'étend depuis la partie d'engagement de pointe (54) de l'insert d'étanchéité (46) dans une direction distale et a une extrémité distale (70) qui correspond à une extrémité distale (52) de l'insert d'étanchéité (46).

5. Capuchon d'étanchéité (10) selon l'une des revendications 1 à 4, dans lequel la nervure de verrouillage (78) est espacée de l'extrémité proximale (50) de l'insert d'étanchéité (46) de sorte qu'une partie de la partie d'engagement de pointe (54) s'étende de manière proximale au-delà de la nervure de verrouillage (78).

6. Capuchon d'étanchéité (10) selon l'une des revendications 1 à 5, dans lequel la nervure de verrouillage (78) a une face périphérique externe qui est inclinée.

7. Capuchon d'étanchéité (10) selon l'une des revendications 1 à 6, dans lequel la nervure de verrouillage (78) a une section transversale trapézoïdale.

8. Capuchon d'étanchéité (10) selon l'une des revendications 1 à 7, dans lequel le diamètre externe (A) de la partie d'engagement de boîtier (56) est supérieur au diamètre externe (B) de la partie d'engagement de pointe (54), et la nervure de verrouillage (78) a un diamètre externe (L) qui est intermédiaire entre le diamètre externe (B) de la partie d'engagement de pointe (54) et le diamètre externe (A) de la partie d'engagement de boîtier (56).

9. Capuchon d'étanchéité (10) selon l'une des revendications 1 à 8, dans lequel la partie d'engagement d'insert (90) a une extrémité proximale ouverte (92), une extrémité distale (94) et une paroi latérale (96) définissant une cavité (98) adaptée et dimensionnée pour recevoir la partie d'engagement de boîtier (56).

10. Capuchon d'étanchéité (10) selon l'une des revendications 1 à 9, dans lequel la saillie annulaire (100) s'étend radialement vers l'intérieur autour de la surface interne de la paroi latérale (96) de la partie d'engagement d'insert (90).

11. Capuchon d'étanchéité (10) selon l'une des revendications 1 à 10, dans lequel la partie de connexion (88) s'étend de manière proximale depuis la partie d'engagement d'insert (90) et comprend une extrémité proximale ouverte (112), une extrémité distale ouverte (114), une paroi latérale (116) définissant une cavité (118) adaptée pour recevoir au moins une partie de la partie d'engagement de pointe (54), et un filetage (120) s'étendant autour d'une surface périphérique externe de la paroi latérale (116) de la partie de connexion (88).

12. Capuchon d'étanchéité (10) selon l'une des revendications 1 à 11, dans lequel le diamètre externe de la partie de connexion (88) est inférieur au diamètre externe de la partie d'engagement d'insert (90).

13. Capuchon d'étanchéité (10) selon l'une des revendications 1 à 12, dans lequel le diamètre de la cavité (98) du boîtier (48) qui reçoit la partie d'engagement de pointe (54) est supérieur au diamètre externe de la partie d'engagement de pointe (54), de sorte qu'un espace soit prévu entre la partie d'engagement de pointe (54) et le boîtier (48).

14. Capuchon d'étanchéité (10) selon l'une des revendications 1 à 13, dans lequel la saillie annulaire (100) est espacée d'une distance (J) de l'extrémité distale (86) du boîtier (48) qui est approximativement supérieure ou égale à une distance (K) entre la rainure annulaire (72) et une extrémité distale (52) de l'insert d'étanchéité (46).

15. Capuchon d'étanchéité (10) selon l'une des revendications 1 à 14, dans lequel un diamètre (F) de la cavité (118) du boîtier (48) au niveau de la saillie annulaire (100) est approximativement égal à un diamètre (G) de l'insert d'étanchéité (46) à l'intérieur de la rainure annulaire (72).

16. Capuchon d'étanchéité (10) selon l'une des revendications 1 à 15, dans lequel la nervure de verrouillage (78) est espacée d'une distance (H) de la rainure annulaire (72) qui est approximativement égale à une distance (I) entre la saillie annulaire (100) et l'extrémité proximale (84) du boîtier (48).

17. Capuchon d'étanchéité (10) selon l'une des revendications 1 à 16, comprenant en outre une pluralité de nervures anti-rotation (124) s'étendant axialement dans une direction longitudinale le long d'une surface périphérique externe de la partie d'engagement de boîtier (56) et/ou une pluralité de nervures anti-rotation (126) s'étendant axialement dans une direction longitudinale le long d'une surface périphérique interne de la partie d'engagement d'insert (90).

18. Capuchon d'étanchéité (10) selon la revendication 17, dans lequel les nervures anti-rotation (124) de la partie d'engagement de boîtier (56) sont reçues dans les espaces entre les nervures anti-rotation (126) de la partie d'engagement d'insert (90).

19. Capuchon d'étanchéité (10) selon l'une des revendications 17 et 18, dans lequel une extrémité proximale (130) des nervures anti-rotation (124) de la partie d'engagement de boîtier (56) et/ou une extrémité distale (128) des nervures anti-rotation (126) de la partie d'engagement d'insert (90) sont effilées ou pointues.

20. Capuchon d'étanchéité (10) selon l'une des revendications 1 à 19, dans lequel un matériau du boîtier (48) a un module de Young plus élevé, une résistance plus élevée, une dureté plus élevée et/ou une élasticité plus faible qu'un matériau de l'insert d'étanchéité (46).

21. Capuchon d'étanchéité (10) selon l'une des revendications 1 à 20, dans lequel l'insert d'étanchéité (46) est réalisé en un matériau choisi dans le groupe constitué par le polyéthylène (PE), le polypropylène (PP), un matériau élastomère, et le boîtier (48) est réalisé en un matériau choisi dans le groupe constitué par le polycarbonate (PC), le polyoxyméthylène (POM), le polybutylène téréphtalate (PBT), le polypropylène (PP), le polyéthylène (PE), le polychlorure de vinyle (PVC), le polystyrène, l'acrylonitrile butadiène styrène, le styrène-acrylonitrile, un polymère renforcé de fibres, un métal et des combinaisons de ceux-ci.

22. Seringue (12) comprenant :
un cylindre de seringue (14) définissant un réservoir (22) pour contenir un médicament ;
une pointe distale (24) ayant un passage (36) en communication fluidique avec le réservoir (22) ; et
un capuchon d'étanchéité (10) selon l'une des revendications 1 à 21,
dans laquelle le capuchon d'étanchéité (10) est configuré pour recouvrir une extrémité distale (44) du passage (26) et pour créer une étanchéité entre l'insert d'étanchéité (46) et une surface externe de la pointe distale (24) afin d'empêcher l'air et les contaminants de pénétrer dans la cavité (98, 118) du capuchon d'étanchéité (10) et/ou le passage (26).

23. Seringue (12) selon la revendication 22, dans laquelle la partie de connexion (88) est engagée par filetage avec un collier Luer (122) entourant une extrémité proximale (28) de la pointe distale (24), et la pointe distale (24) est reçue dans une cavité (64) de la partie d'engagement de pointe (54) de l'insert d'étanchéité (46).

24. Seringue (12) selon la revendication 23, dans laquelle le collier Luer (122) est un composant distinct qui est ajusté par friction sur la pointe distale (24).

25. Seringue (12) selon la revendication 24, dans laquelle le collier Luer (122) comprend une extrémité proximale (132) adjacente à une face distale de la seringue (12), une extrémité distale ouverte (134), et une paroi latérale (136) espacée de la pointe distale (24) et entourant au moins partiellement celle-ci, et une surface interne de la paroi latérale (136) du collier Luer (122) comprend un filetage (138) qui est engagé par un filetage (120) sur une surface externe de la partie de connexion (88) et une pluralité de protubérances de montage dirigées radialement vers l'intérieur (140) s'étendent depuis la paroi latérale (136) du collier Luer (122) au niveau de l'extrémité proximale (132) du collier Luer (122) et s'engagent par friction avec la pointe distale (24).
